# EUROPEAN PATENT SPECIFICATION

(11) **EP 2 987 533 B1**
(45) Date of publication and mention of the grant of the patent: **11.07.2018**
(21) Application number: 14181731.2
(22) Date of filing: 21.08.2014
(51) Int. Cl.: A61P 35/00, A61K 31/496, A61K 31/5377, A61K 31/549, C07D 215/56, C07D 401/12

(54) **7-substituted piperazin-1-yl fluoroquinolone compounds, their use in the treatment of cancer, pharmaceutical compositions and a method for preparation**
7-substituierte Piperazin-1-yl-Fluorchinolonverbindungen, deren Verwendung bei der Behandlung von Krebs, pharmazeutische Zusammensetzungen und Verfahren zur Herstellung
Piperazin-1-yl fluoroquinolones substituées en position 7, leur utilisation dans le traitement du cancer, compositions pharmaceutiques et méthodes de préparation

(43) Date of publication of application: 24.02.2016
(73) Proprietor: King Saud University, 11421 Riyadh (SA)
(72) Inventor: Abdel-Aziz, Alaa A.-M., 11333 Riyadh (SA); El-Azab, Adel S., 11333 Riyadh (SA); Alanazi, Amer M., 11333 Riyadh (SA); Asiri, Yousif A., 11333 Riyadh (SA); Al-Suwaidan, Ibrahim A., 11333 Riyadh (SA)
(74) Representative: Goddar, Heinz J.

(56) References cited:
- WO-A1-2007/059195
- ES-A6- 2 006 098
- RO-B1- 123 352
- US-A- 5 395 936
- US-A1- 2004 132 764
- CASTRO W ET AL: "Medicinal potential of ciprofloxacin and its derivatives", FUTURE MEDICINAL CHEMISTRY, FUTURE SCIENCE LTD, GB, vol. 5, no. 1, 1 January 2013 (2013-01-01) , pages 81-96, XP009180459, ISSN: 1756-8919, DOI: 10.4155/FMC.12.181
- ABDEL-AZIZ MOHAMED ET AL: "Novel N-4-piperazinyl-ciprofloxacin-chalcone hybrids: Synthesis, physicochemical properties, anticancer and topoisomerase I and II inhibitory activity", EUROPEAN JOURNAL OF MEDICINAL CHEMISTRY, vol. 69, 12 September 2013 (2013-09-12), pages 427-438, XP028762815, ISSN: 0223-5234, DOI: 10.1016/J.EJMECH.2013.08.040
- AZÉMA J. ET AL.: BIOORG. MED CHEM., vol. 17, 2009, pages 5396-5407, XP26336548,
- SHI Z -Y ET AL: "Piperonal ciprofloxacin hydrazone induces growth arrest and apoptosis of human hepatocarcinoma SMMC-7721 cells", ACTA PHARMACOLOGICA SINICA 2012 NATURE PUBLISHING GROUP GBR, vol. 33, no. 2, February 2012 (2012-02), pages 271-278, XP55144380, ISSN: 1671-4083
- MOHAMMAD SHAHARYAR ET AL: "Synthesis and antiproliferative activity of 1-[(sub)]-6-fluoro-3-[(sub)]-1, 3,4-oxadiazol-2-yl-7-piperazino-1, 4-dihydro-4-quinolinone derivatives", MEDICINAL CHEMISTRY RESEARCH, BIRKHÄUSER-VERLAG, BO, vol. 16, no. 6, 23 October 2007 (2007-10-23), pages 292-299, XP019590664, ISSN: 1554-8120
- SURESH NARVA ET AL: "Synthesis of novel ciprofloxacin analogues and evaluation of their anti-proliferative effect on human cancer cell lines", BIOORGANIC & MEDICINAL CHEMISTRY LETTERS, vol. 23, no. 23, 1 October 2013 (2013-10-01), pages 6292-6295, XP028760141, ISSN: 0960-894X, DOI: 10.1016/J.BMCL.2013.09.077
- ALAA A-M ABDEL-AZIZ ET AL: "Design, synthesis and antibacterial activity of fluoroquinolones containing bulky arenesulfonyl fragment: 2D-QSAR and docking study", EUROPEAN JOURNAL OF MEDICINAL CHEMISTRY, EDITIONS SCIENTIFIQUE ELSEVIER, PARIS, FR, vol. 46, no. 11, 7 September 2011 (2011-09-07), pages 5487-5497, XP028324210, ISSN: 0223-5234, DOI: 10.1016/J.EJMECH.2011.09.011 [retrieved on 2011-09-16]
- NIETO M J ET AL: "Benzenesulfonamide analogs of fluoroquinolones. Antibacterial activity and QSAR studies", EUROPEAN JOURNAL OF MEDICINAL CHEMISTRY, EDITIONS SCIENTIFIQUE ELSEVIER, PARIS, FR, vol. 40, no. 4, 1 April 2005 (2005-04-01), pages 361-369, XP027857743, ISSN: 0223-5234 [retrieved on 2005-04-01]
- F. ALOVERO: "Comparative study of new benzenesulphonamide fluoroquinolones structurally related to ciprofloxacin against selected ciprofloxacin-susceptible and -resistant Gram-positive cocci", JOURNAL OF ANTIMICROBIAL CHEMOTHERAPY, vol. 48, no. 5, 1 November 2001 (2001-11-01), pages 709-712, XP055143357, DOI: 10.1093/jac/48.5.709
- DATABASE CA [Online] CHEMICAL ABSTRACTS SERVICE, COLUMBUS, OHIO, US; 23 April 2003 (2003-04-23), QU, LINGBO ET AL: "Studies on the synthesis and antibacterial activity of quinolone analogues", XP7922853, retrieved from STN Database accession no. 2003:311128
- RAVEENDRA DAYAM ET AL: "Discovery of small molecule integrin alpha(v)beta(3) antagonists as novel anticancer agents", JOURNAL OF MEDICINAL CHEMISTRY, AMERICAN CHEMICAL SOCIETY, US , vol. 49, no. 15 1 January 2006 (2006-01-01), pages 4526-4534, XP002674944, ISSN: 0022-2623, DOI: 10.1021/JM051296S Retrieved from the Internet: URL:http://pubs.acs.org/doi/abs/10.1021/jm 051296s [retrieved on 2006-07-01] & Raveendra Dayam ET AL: "Discovery of Small Molecule Integrin [alpha] v [beta] 3 Antagonists as Novel Anticancer Agents - Supporting Information", JOURNAL OF MEDICINAL CHEMISTRY, AMERICAN CHEMICAL SOCIETY, US, 1 January 2006 (2006-01-01), XP055143363, Retrieved from the Internet: URL:http://pubs.acs.org/doi/suppl/10.1021/ jm051296s/suppl_file/jm051296ssi20060526_0 64248.pdf [retrieved on 2014-09-29]
- SURESH NARVA ET AL: "Synthesis and evaluation of 1-cyclopropyl-6-fluoro-1,4-dihydro-4-oxo-7 -(4-(2-(4-substitutedpiperazin-1-yl)acetyl )piperazin-1-yl)quinoline-3-carboxylic acid derivatives as anti-tubercular and antibacterial agents", EUROPEAN JOURNAL OF MEDICINAL CHEMISTRY, EDITIONS SCIENTIFIQUE ELSEVIER, PARIS, FR, vol. 71, 20 November 2013 (2013-11-20), pages 324-332, XP028829692, ISSN: 0223-5234, DOI: 10.1016/J.EJMECH.2013.10.055
- EL-DIN GAD ET AL: "Design, synthesis, antibacterial activity and physicochemical parameters of novel N-4-piperazinyl derivatives of norfloxacin", BIOORGANIC & MEDICINAL CHEMISTRY, PERGAMON, GB, vol. 17, no. 11, 1 June 2009 (2009-06-01), pages 3879-3886, XP026118935, ISSN: 0968-0896, DOI: 10.1016/J.BMC.2009.04.027 [retrieved on 2009-04-19]
- PO-TING CHEN ET AL: "New 7-[4-(4-(un)Substituted)piperazine-1-carbo nyl]- piperazin-1-yl] Derivatives of Fluoroquinolone: Synthesis and Antimicrobial Evaluation", MOLECULES, vol. 18, no. 7, 27 June 2013 (2013-06-27), pages 7557-7569, XP055164520, DOI: 10.3390/molecules18077557
- DATABASE CA [Online] CHEMICAL ABSTRACTS SERVICE, COLUMBUS, OHIO, US; 10 October 2011 (2011-10-10), GIRIJA, K. ET AL: "Ecofriendly synthesis and biological evaluation of some novel N-substituted piperazinyl fluoro quinolone derivatives", XP002735126, retrieved from STN accession no. 2011:1279046 Database accession no. 2011:1279046

## Description

The present invention relates to 7-substituted piperazin-1-yl fluoroquinolone compounds, their use in the treatment of cancer, pharmaceutical compositions and a method for the preparation.

Cancer is continuing to be a major health problem worldwide to treat and is the leading cause of human mortality exceeded only by cardiovascular diseases. The development of a novel and efficient anticancer agents remains an important and challenging goal in medicinal chemistry. Therefore, developing new anticancer drugs and more effective treatment strategies for cancer with well-defined pharmacokinetic properties are of importance (Avendaño C., Menéndez J.C., Medicinal Chemistry of Anticancer Drugs; Elsevier: Amsterdam, Netherlands, (2008), pages: 1-459). Quinolone antibiotics come close to be ideal chemotherapeutic agents in that they are administered orally, concentrated in cells and tissues, and readily available. Quinolones are known for their antimicrobial and antitumor activities through DNA intercalation and alteration of the normal functions of bacterial gyrase, and were found to be a topoisomerase II inhibitor in humans (Azéma J. et al., Bioorg. Med. Chem. 2009, 17, 5396-5407; Yamashita Y. et al., Cancer Research 52, 1992, 2818-2822). Ciprofloxacin, a commonly used broad-spectrum fluoroquinolone antibiotic with low side effects, has been shown to have antiproliferative and apoptotic activities in several cancer cell lines (Azéma J. et al., Bioorg. Med. Chem. 2009, 17, 5396-5407; Paul M. et al., Eur. J. Clin. Microbiol. Infect. Dis. 2007, 26, 825-831). In addition, ciprofloxacin and some of its derivatives have shown to be effective against other diseases as malaria and AIDS (Biot C. et al., Future Science 2013, 5, 81-96). Voreloxin is a quinolone derivative that has no antibacterial activity, but shows potent cytotoxicity towards eukaryotic cancer cell lines. This compound intercalates DNA and inhibits topoisomerase II and it is currently being evaluated in a Phase 2 clinical trial for resistant ovarian cancer (Advani R.H. et al., Clin. Cancer 2010, 16, 2167-2175; Hawtin R.E. et al., PLoS ONE, 2010, 5, e10186).

An antibacterial agent that is active against both gram-negative and gram-positive bacteria (Abdel-Aziz et al., Eur. J. Med. Chem. 2011, 46, 5487-5497; Nieto M.J. et al., Eur. J. Med. Chem. 2005, 40, 361-369; US 5,395,936A) can be used as a single antibiotic for prophylaxis as well as treatment of bacterial super-infections in cancer patients receiving chemotherapy.

Obviously, if such an agent is also effective in preventing the growth of tumor cells, it is a dual impact.

WO 2007/059195 A1 discloses a composition containing integrin-binding small molecules.

Castro et al., Future Medicinal Chemistry, 2013, 5(1), 81 deals with the medicinal potential of ciprofloxacin and its derivatives.

Abdel-Aziz, European Journal of Medicinal Chemistry, 2013, 69, 427 discloses N-4-piperazinyl-ciprofloxacin-chalcone hybrids and discusses anticancer properties thereof.

Azéma et al., Bioorganic and Medicinal Chemistry, 2009, 17,5396 discloses 7-(4-substituted) piperacin-1-yl) derivatives of ciprofloxacin and investigates the potential thereof as antitumor agent.

Shi et al., Acta Pharmacologica Sinica, 2012, 33, 271 discloses piperonal ciprofloxacin hydrazone induced growth arrest and apoptosis of human hepatocarcinoma SMMC-7721 cells.

Shaharyar et al., Medicinal Chemistry Research, 2007, 16 (6), 292 discloses synthesis and anti-proliferative activity of 1-[(sub)]-6-fluoro-3-[(sub)]-1,3,4-oxadiazol-2-yl-7-piperazino-1, 4-dihydro-4-quinolinone derivatives.

Narva et al., Bioorganic and Medicinal Chemistry Letters, 2013, 23 (23), 6292 discloses the synthesis of novel ciprofloxacin analogues and evaluation of their anti-proliferative effect on human cancer cell lines.

ES 2 006 098 A6 discloses ciprofloxacin hydrocholoride monohydrate preparation.

RO 123 352 B1 discloses fluoroquinolone piperacinyl sulfonyl phenoxyacetic derivatives and a process for preparing the same.

US 5 395 936 A discloses 7-(4-[4-aminophenyl)sulfonyl]-1-piperacinyl fluoroquinolonique derivatives and the synthesis thereof.

Abdel-Aziz, European Journal of Medicinal Chemistry, 2011, 46 (11), 5487 discloses design, synthesis and antibacterial activity of fluoroquinolones containing bulky arenesulfonyl fragments.

Nieto et al., European Journal of Medicinal Chemistry, 2005, 40 (4), 361 discloses benzenesulfonamide analogs of fluoroquinolones and antibacterial behavior thereof.

Alovero, Journal of Antimicrobial Chemotherapy, 2001, 48 (5), 709, discloses comparative studies of new benzenesulphonamide fluoroquinolones structurally related to ciprofloxacin against selected Gram-positive bacteria.

Lingbo et al., Chemical Abstract Service, 23. April 2003 discloses studies on the synthesis and antibacterial activity of quinolone analogues.

Dayam et al., Journal of Medicinal Chemistry, 2006, 49 (15), 4526 discloses a discovery of small molecule integrin alpha beta antagonists as novel anticancer agents.

It is an object of the present invention to provide novel and antitumor active 7-substituted piperazin-1-yl fluoroquinolone derivatives which overcome the drawbacks of the prior art and can be utilized in the treatment of cancer. Especially, compounds shall be provided which exhibit potent and extensive antitumor activities without abolishing their inherent antibacterial activities and thereby reducing the need in using several compounds in combined antitumor and antibacterial treatment for cancer patient with concurrent bacterial secondary infection. It is a further object to provide a novel and efficient method for the preparation of 7-substituted piperazin-1-yl fluoroquinoline derivatives which ensures fast and simple access to respective derivatives having general formulas (II) and (III).

The above object is achieved in accordance with the independent claim.

In particular the object is achieved by a compound of general formula (I): 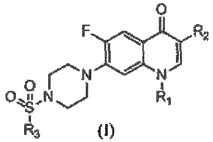 wherein R₁ is cyclopropyl; R₂ is a carboxyl group; and; R₃ is selected from 4-chlorophenyl, 2,4-dichlorophenyl, 3,4-dichlorophenyl, 3,4-dimethoxyphenyl, 2,4-dimethoxyphenyl, and 2,4,6-trimethylphenyl; or pharmaceutically acceptable salt thereof for use in treating cancer.

Further described herein is a compound of general formulas (I), (II) or (III): 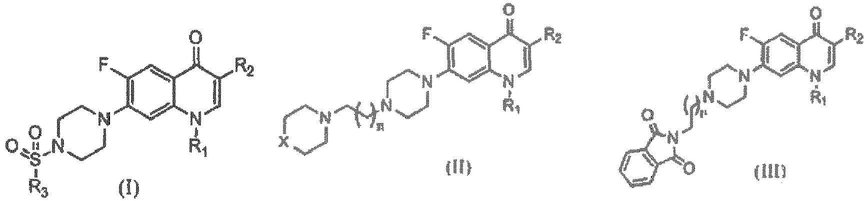
wherein R₁ is ethyl or cyclopropyl;
R₂ is a carboxyl group; an ester group having 1 to 4 carbon atoms; a hydrazide, C₁-C₁₀-alkylhydrazide or phenylhydrazide group each optionally substituted with one or two substituents selected from C₁-C₁₀-alkyl, halogen, C₁-C₁₀-alkoxy, nitro and/or amino; a diacyl hydrazide, diacyl C₁-C₁₀-alkylhydrazide or diacyl phenylhydrazide group each optionally substituted with one or two substituents selected from C₁-C₁₀-alkyl, halogen, C₁-C₁₀-alkoxy, nitro and/or amino; or an oxadiazole group optionally substituted with C₁-C₁₀-alkyl, C₁-C₁₀-cycloalkyl or C₆-C₂₀-aryl group, wherein the alkyl, cycloalkyl or aryl group are optionally substituted with one or two substituents selected from C₁-C₁₀-alkyl, halogen, C₁-C₁₀-alkoxy, nitro and/or amino;
R₃ is C₁-C₁₀-alkyl or C₆-C₂₀-aryl group, wherein the alkyl or aryl group are optionally substituted with 1 to 3 substituents selected from C₁-C₁₀-alkyl, halogen, of C₁-C₁₀-alkoxy, nitro and/or amino;
X is a carbon atom or a heteroatom of O, S, N; and
n is 0, 1 or 2;
or pharmaceutically acceptable salt thereof for use in treating cancer.

The compound of general formula (I) for use in treating cancer may have a structure of formula (Id): 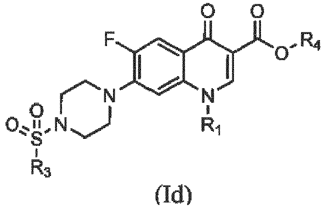 wherein R₃ is selected from the group of 3,4-dimethoxyphenyl, 2,4-dimethoxyphenyl, 3,4-dichlorophenyl, 2,4-dichlorophenyl, 2,4,6-dichlorophenyl, 2,4-difluorophenyl, 2,4,6-trimethylphenyl, 2,3,4,5,6-pentamethylphenyl and 2,4,6-triisopropylphenyl; and
R₄ is hydrogen, methyl, ethyl, propyl, isopropyl or butyl.

The compound of general formula (I) for use in treating cancer may have a structure of formulas (Ia), (Ib) or (Ic): 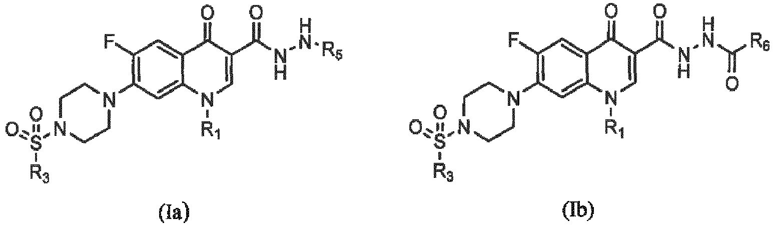 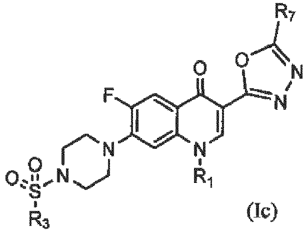 wherein R₅, R₆ and R₇ are selected from the group consisting of hydrogen, methyl, isopropyl, butyl, 1-adamantyl, cyclohexyl, unsubstituted or substituted phenyl and unsubstituted or substituted benzyl.

Further described herein is a pharmaceutical composition comprising at least one compound of general formulas (I), (II) or (III) and/or pharmaceutically acceptable salt thereof for use in the treatment of cancer.

The term "pharmaceutical composition", as used herein, is intended to comprise at least one pharmaceutical active compound of general formulas (I), (II) or (III) and/or a pharmaceutical acceptable salt thereof.

The pharmaceutical composition can be, for example, in a liquid form, e.g. a solution, syrup, emulsion and suspension, or in a solid form, e.g. a capsule, caplet, tablet, pill, powder and suppository. Granules, semi-solid forms and gel caps are also considered. In case that the pharmaceutical composition is a liquid or a powder, dosage unit optionally is to be measured, e.g. in the dosage unit of a teaspoon. In addition to the compound of general formulas (I), (II) or (III), the pharmaceutical composition can comprise, for example, flavoring agents, sweeteners, dyes, stabilizers, diluents, suspending agents, granulating agents, lubricants, binders and disintegredient agents. A tablet, for example, can be coated. All of the formulations mentioned can be intended for immediate-release, timed release and sustained release.

All components of the pharmaceutical composition have to be pharmaceutically acceptable. The term "pharmaceutically acceptable" means at least non-toxic. The therapeutically active component should preferably be present in the above-mentioned pharmaceutical composition, the concentration of about 0.1 to 99.5% by weight, preferably of about 0.5 to 95% by weight of the total mixture.

The above-mentioned pharmaceutical composition can further contain other pharmaceutical active compounds in addition to the compound of general formulas (I), (II) or (III) according to the invention.

Further described herein is the use of a compound of general formulas (I), (II) or (III) and/or pharmaceutically acceptable salt thereof, in the manufacture of a medicament for the treatment of cancer.

Further described herein is a compound of general formulas (I), (II) or (III), wherein in compound of general formula (I), R₂ is not a carboxyl group.

Further described herein is a method for preparing a compound of general formulas (II) or (III), comprising the step of reacting ciprofloxacin or norfloxacin with heterocyclic alkyl halide or phthalamide alkyl halide fragments, preferably *N*-ethylphthalimide bromide, N-propylphthalimide bromide, chloroethylpiperidine, chloroethylmorpholine, chloroethylpyrrolidine, 2-chloro-N-(4-sulfamoylphenyl)acetamide, 2-chloro-N-(4-sulfamoylbenzyl)acetamide, 3-chloro-N-(4-sulfamoylphenyl)propanamide, 3-chloro-N-(4-sulfamoylbenzyl)propanamide, 2-chloro-N-phenylacetamide, substituted 2-chloro-N-phenylacetamide, 3-chloro-N-(4-ethylphenyl)propanamide or substituted 3-chloro-N-(4-ethylphenyl)propanamide.

Surprisingly, it was found that a compound in accordance with claim 1 exhibits potential antitumor activity within 60 human cell lines and additionally keeping its inherent antibacterial activity. Therefore, the compound cannot only be used in cancer treatment, but also in combined antitumor and antibacterial treatment for cancer patients with concurrent bacterial secondary infections. Moreover, the method for preparing compounds having general formulas (II) and (III) provides the possibility to synthesize antiturmor active compounds in a simple and time-saving way starting from ciprofloxacin and norfloxacin as scaffolds. Apart from the above-mentioned features, the inventive compound shows a good crystallinity and a good solubility in most of the solvents.

Compounds of general formula (I) can be prepared by the following scheme:

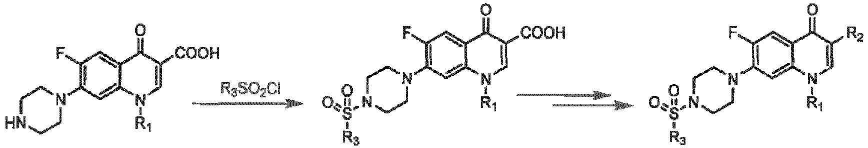

For the synthesis of compounds having general formula (I), ciprofloxacin or nofloxacin are first reacted with arenesulfonyl chloride under basic condition to form a 7-(4-sulfonyl)piperazin-1-yl)-3-carboxylic acid derivative (see Abdel-Aziz et al., E.J. Med. Chem. 2011, 46, 5487-5497). The arenesulfonyl chloride may be, but is not limited to, 3,4-dimethoxybenzenesulfonyl chloride, 2,4-dimethoxybenzenesulfonyl chloride, 3,4-dichlorobenhenesulfonyl chloride, 2,4-dichlorobenzenesulfonyl chloride, 2,4,6-trichlorobenzenesulfonyl chloride, 2,4-difluorobenzenesulfonyl chloride, 2,4,6-dimethylbenzenesulfonyl chloride, 2,3,4,5,6-pentamethylbenzenesulfonyl chloride or 2,4,6-triisopropylbenzenesulfonylchloride. For the reaction, the sulfonyl compound is dissolved in alcohol including, but is not limited to, methanol, ethanol, isopropanol and butanol. To form further compounds having general formula (I), the 3-carboxylic acid derivative may be subjected to several reactions starting from conversion of the acid into the corresponding ester. The reaction may be carried out under acidic conditions to afford the ester of formula (Id), wherein R₄ may be, but is not limited to, methyl, ethyl, propyl, isopropyl or butyl. The ester in turn can be transformed to the acid hydrazide derivatives using unsubstituted or substituted hydrazine or hydrazide including, but is not limited to, hydrazine hydrate, methylhydrazine, cyclohexylhydrazine, phenylhydrazine, 4-methoxyhydrazine, 4-chlorohydrazine, 4-methylhydrazine, 4-fluorophenylhydrazine, acetic acid hydrazide, 1-adamantane carboxylic acid hydrazide, cyclohexane carboxylic acid hydrazide, benzoic acid hydrazide, 4-methoxybenzoic acid hydrazide, 3,4-dimethoxybenzoic acid hydrazide, 4-chlorobenzoic acid hydrazide, 4-methylbenzoic acid hydrazide or vanillic acid hydrazide. The acid hydrazide or diacyl hydrazide may then be converted to the corresponding oxadiazole having formula (Ic) by acid cyclisation. The acid environment may be created by adding an acid component including, but not limited to, H₂SO₄, POCl₃ and Lewis acid.

The invention is now further illustrated on the basis of Examples and a detailed description from which further features and advantages may be taken. It is to be noted that the following explanations are presented for the purpose of illustrating and description only; they are not intended to be exhaustive or to limit the invention to the precise form disclosed.

### Example 1

### General method for synthesis of ciprofloxacin and norfloxacin containing 2-morpholinoethyl and 2-(piperidin-1-yl)ethyl fragments.

A mixture of norfloxacin or ciprofloxacin (1 mmol) and K₂CO₃ (1.1 mmol) was stirred in DMF (10 mL) at room temperature for 20 min. To the resulted mixture, the 1-(2-chloroethyl)piperidine or 4-(2-chloroethyl)morpholine (1.1 mmol) in DMF (5 mL) was added dropwise over a period of 10 min. The reaction mixture was further stirred at room temperature for 24 h. The separated solid was then filtered, washed with cold water, dried and crystallized from the appropriate solvent.

### 1-Cyclopropyl-6-fluoro-7-(4-(2-morpholinoethyl)piperazin-1-yl)-4-oxo-1,4-dihydroquinoline-3-carboxylic:

Yield, 66%; mp 145-147°C (MeOH/CH₂Cl₂); IR(KBr) ν max/cm⁻¹: 3441 (OH),1733 (C=O), 1628 (C=O). ¹HNMR (CDCl₃): δ 1.16 (2H, s), 1.35-1.36 (2H, d, *J* = 6.0 Hz), 2.61 (4H, s), 2.80-2.82 (2H, t, *J* = 7.5 Hz), 3.24 (2H, s), 3.30 (2H, s), 3.45 (1H, s), 3.63 (2H, s), 3.74 (4H, s), 3.78 (2H, s), 4.46-4.47 (2H, d, *J* = 6.0 Hz), 7.28 (1H, s), 8.15 (1H, s), 8.56 (1H, s). ¹³C NMR (CDCl₃): δ 8.2, 34.5, 39.7, 45.4, 49.4, 51.0, 53.9, 57.0, 62.0, 63.4, 66.9, 105.3, 110.3, 113.6, 113.7, 123.8, 137.9, 144.0, 148.4, 152.4, 160.7, 165.6, 173.0. MS m/z (%): 444.5 (3.8%).

### 1-Cyclopropyl-6-fluoro-4-oxo-7-(4-(2-(piperidin-1-yl)ethyl)piperazin-1-yl)-1,4-dihydroquinoline-3-carboxylic acid:

Yield, 73%; mp 265-266°C (MeOH/CH₂Cl₂). ¹HNMR (CDCl₃/CD₃OD): δ 1.23 (2H, s), 1.44-1.45 (2H, d, *J* = 6.0 Hz), 1.71 (2H, s), 1.92 (4H, s), 2.80 (4H, s), 2.87 (2H, s), 3.28-3.29 (6H, d, *J* = 5.5 Hz), 3.45 (4H, s), 3.76 (1H, s), 7.54-7.55 (1H, d, *J* = 7.0 Hz), 7.86-7.89 (1H, d, *J* = 13.0 Hz), 8.77 (1H, s). ¹³C NMR (CDCl₃/CD₃OD): δ 8.8, 22.9, 24.2, 37.0, 50.7, 53.6, 53.9, 54.5, 54.9, 107.0, 108.3, 112.6, 112.8, 140.7, 149.2, 169.6. MS m/z (%): 442.5 (18.4%).

### 1-Ethyl-6-fluoro-4-oxo-7-(4-(2-(piperidin-1-yl)ethyl)piperazin-1-yl)-1,4-dihydroquinoline-3-carboxylic acid:

Yield, 69%; mp 259-260°C (MeOH/CH₂Cl₂); IR(KBr) v max/cm⁻¹: 3440 (OH),1730 (C=O), 1636 (C=O). ¹HNMR (CDCl₃/CD₃OD): δ 1.56-1.59 (3H, t, *J* = 7.0 Hz), 1.71 (2H, s), 1.94 (4H, s), 2.79 (4H, s), 2.88 (2H, s), 3.30-3.33 (6H, d, *J* = 13.5 Hz), 3.43 (4H, s), 4.51-4.52 (2H, d, *J* = 7.0 Hz), 7.08-7.09 (1H, d, *J* = 6.5 Hz), 7.90-7.93 (1H, d, *J* = 13.5 Hz), 8.80 (1H, s). ¹³C NMR (CDCl₃/CD₃OD): δ 15.2, 22.8, 24.1, 50.8, 51.1, 53.5, 53.9, 54.4, 54.9, 64.8, 106.2, 108.6, 113.0, 113.1, 121.3, 138.8, 147.4, 147.5, 149.2, 154.0, 169.7, 178.2. MS m/z (%): 430.5 (6.8%).

### Example 2

### General method for synthesis of norfloxacin containing phthalimide fragment.

### 7-(4-(3-(1,3-Dioxoisoindolin-2-yl)propyl)piperazin-1-yl)-1-ethyl-6-fluoro-4-oxo-1,4-dihydroquinoline-3-carboxylic acid:

A mixture of norfloxacin (a) (1.0 mmol) and K₂CO₃ (1.1 mmol) was stirred in DMF (10 mL) at room temperature for 20 min. To the resulted mixture, the 2-(2-bromoethyl)isoindoline-1,3-dione or 2-(3-bromopropyl)isoindoline-1,3-dione (1.1 mmol) in DMF (5 mL) was added dropwise over a period of 10 min. The reaction mixture was further stirred at room temperature for 24 h. The separated solid was then filtered, washed with cold water, dried and crystallized from the appropriate solvent.

Yield, 63%; mp 205-207 °C (Hexane/CH₂Cl₂). ¹HNMR (CDCl₃): δ 1.59-1.61 (3H, t, *J* = 7.0 Hz), 1.92-1.94 (2H, t, *J* = 6.5 Hz), 2.54 (2H, s), 2.65 (4H, s), 3.23 (4H, s), 3.82-3.84 (2H, t, *J* = 7.0 Hz), 4.33-4.34 (2H, d, *J* = 7.0 Hz), 6.77-6.78 (1H, d, *J* = 6.5 Hz), 7.72-7.74 (2H, dd, *J* = 5.0, 8.0 Hz), 7.86-7.88 (2H, dd, *J* = 5.5, 8.5 Hz), 8.01-8.04 (1H, d, *J* = 13.0 Hz), 8.68 (1H, s), 15.14 (1H, s, br). ¹³C NMR (CDCl₃): δ 14.4, 25.2, 36.4, 49.7, 52.6, 52.6, 103.6, 108.3, 112.6, 112.8, 120.4, 123.1, 132.2, 133.9, 137.0, 146.0, 146.1, 147.0, 152.5, 154.5, 167.2, 168.5, 176.9. MS m/z (%):506.5 (6.4%).

### Example 3

### Evaluation of the antitumor activity of the synthesized compounds (tables 1, 2, 3, 4 and 5).

A primary anticancer assay was performed for an approximately 60 human tumor cell line panel derived from nine neoplastic diseases, in accordance with the protocol of the Drug Evaluation Branch, National Cancer Institute, Bethesda, MD (Monks A. et al., J. Natl. Cancer Inst 1991, 83, 757-766; Boyd M.R. and Paull K.D., Drug. Dev. Res. 1995, 34, 91-109; Shoemaker R.H., Nat. Rev. Cancer 2006, 6, 813-823). Briefly, tested compounds were added to the culture at a single concentration (10⁻⁵M), and the cultures were incubated for 48 h. End point determinations were made with a protein binding dye, sulforhodamine B (SRB). The results for each tested compound were reported as the growth percentage of the treated cells when compared to that of the untreated control cells. The percentage growth was evaluated spectrophotometrically versus controls not treated with test agents. The cytotoxic and/or growth inhibitory effects of the most active selected compounds were tested *in vitro* against the full panel of about 60 human tumor cell lines at 10-fold dilutions of five concentrations ranging from 10⁻⁴ to 10⁻⁸ M. A 48 h continuous drug exposure protocol was followed, and an SRB protein assay was used to estimate cell viability or growth.

Three dose response parameters (GI₅₀, TGI, and LC₅₀) were calculated for each compound. Growth inhibition of 50% (GI₅₀) was calculated, which was the drug concentration resulting in a 50% lower net protein increase in the treated cells (measured by SRB staining) compared to the net protein increase seen in the control cells. The compounds having GI₅₀ ≤ 100 µM were declared to be active.

The drug concentration resulting in total growth inhibition (TGI) was calculated, which was the concentration of drug resulting in a 50% reduction in the measured protein at the end of the drug treatment compared to that at the beginning.

**Table 1. Antitumor activity of the arenesulfonyl fluoroquinolones at 10µM concentration**

| | | | | |
|---|---|---|---|---|
| 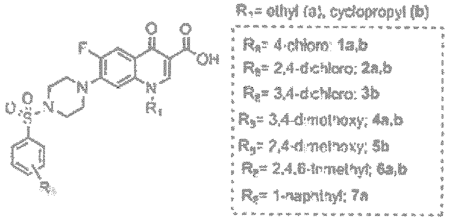 | | | | |

| Compd No | 60 cell lines assay in one dose 10.0 mM concentration | | | |
|---|---|---|---|---|
| | MGI % | PCE | RGI % | Most sensitive cell lines |
| **1a** | 75.59 | 56/56 | >100.0 to 31.3 | *Leukemia* (CCRF-CEM, HL-60 (TB), K-562, MOLT-4, PRMI-8226), *NSC Lung Cancer* (A549/ATCC, NCI-H226, HOP-92, NCI-H23, NCI-H322M, NCI-H460, NCI-H522), *Colon Cancer* (COLO 205, HCC-2998, HCT-116, HCT-15, HT29, KM12, SW-620), *CNS Cancer* (SF-268, SF-295, SF-539, SNB-19,SNB-75, U251), *Melanoma* (LOX IMVI, MALME-3M , M14, MDA-MB-435, SK-MEL-2, SK-MEL-28, SK-MEL-5, UACC-257, UACC-62), *Ovarian Cancer* (IGROV1, OVCAR-3, OVCAR-4, OVCAR-5, OVCAR-8, NCI/ADR-RES, SK-OV-3), *Renal Cancer* (786-0, A498, ACHN, CAKI-1, RXF 393, SN12C, TK-10, UO-31), *Prostate Cancer* (PC-3, DU-145), *Breast Cancer* (MCF7, MDA-MB-231/ATCC , HS 578T, BT-549, T-47D) |
| **1b** | 3.5 | 7/53 | 59.75 to 10.12 | *Melanoma* (LOX IMVI, M14), *Renal Cancer* (A498, CAKI-1, UO-31), *Prostate Cancer* (PC-3), *Breast Cancer* (T-47D) |
| **2a** | 94.17 | 56/58 | >100.0 to 25.22 | *Leukemia* (CCRF-CEM, HL-60(TB), K-562, MOLT-4, PRMI-8226, SR), *NSC Lung Cancer* (A549/ATCC, HOP-62, NCI-H226, HOP-92, NCI-H23, NCI- H322M, NCI-H460, NCI-H522), *Colon Cancer* (COLO 205, HCC-2998, HCT-116, HCT-15, HT29, KM12, SW-620), *CNS Cancer* (SF-268, SF-295, SF-539, SNB-19,SNB-75, U251), *Melanoma* (LOX IMVI, MALME-3M, M14, MDA-MB-435, SK-MEL-2, SK-MEL-5, UACC-257, UACC-62), *Ovarian Cancer* (IGROV1, OVCAR-3, OVCAR-5, OVCAR-8, NCI/ADR-RES, SK-OV-3), *Renal Cancer* (786-0, A498, ACHN, CAKI-1, RXF 393, SN12C, TK-10, UO-31), *Prostate Cancer* (PC-3, DU-145), *Breast Cancer* (MCF7, MDA-MB-231/ATCC , BT-549, T-47D, MDA-MB-468) |
| **2b** | 17.39 | 30/58 | 79.75 to 11.78 | *Leukemia* (CCRF-CEM, HL-60(TB), MOLT-4, PRMI-8226, SR), *NSC Lung Cancer* (HOP-62, NCI-H226, NCI-H23, NCI-H322M), *Colon Cancer* (COLO 205, HCT-116, HCT-15), *CNS Cancer* (SF-268, SNB-75), *Melanoma* (LOX IMVI, SK-MEL-28, SK-MEL-5, UACC-62), *Ovarian Cancer* (OVCAR-4), *Renal Cancer* (A498, ACHN, CAKI-1, RXF 393, SN12C, UO-31), *Prostate Cancer* (PC-3), *Breast Cancer* (MCF7, BT-549, T-47D, MDA-MB-468) |
| **3b** | 89.25 | 58/58 | >100.0 to 46.49 | *Leukemia* (CCRF-CEM, HL-60(TB), K-562, MOLT-4, PRMI-8226, SR), *NSC Lung Cancer* (A549/ATCC, HOP-62, NCI-H226, HOP-92, NCI-H23, NCI-H322M, NCI-H460, NCI-H522), *Colon Cancer* (COLO 205, HCC-2998, HCT-116, HCT-15, HT29, KM12, SW-620), *CNS Cancer* (SF-268, SF-295, SF-539, SNB-19,SNB-75, U251), *Melanoma* (LOX IMVI, MALME-3M, M14, MDA-MB-435, SK-MEL-2, SK-MEL-28, SK-MEL-5, UACC-257, UACC-62), *Ovarian Cancer* (IGROV1, OVCAR-3, OVCAR-4, OVCAR-5, OVCAR-8, NCI/ADR-RES, SK-OV-3), *Renal Cancer* (786-0, A498, ACHN, CAKI-1, RXF 393, SN12C, TK-10, UO-31), *Prostate Cancer* (PC-3, DU-145), *Breast Cancer* (MCF7, MDA-MB-231/ATCC, BT-549, T-47D, MDA-MB-468) |
| **4a** | 2.33 | 9/58 | 55.93 to 10.0 4 | *Leukemia* (MOLT-4, SR), *CNS Cancer* (SF-268, SNB-75) *Melanoma* (LOX IMVI, M14), *Renal Cancer* (UO-31), *Prostate Cancer* (PC-3), *Breast Cancer* (T-47D) |
| **4b** | 1.84 | 6/58 | 35.09 to 10.43 | *NSC Lung Cancer* (HOP-62, HOP-92), *CNS Cancer* (SNB-75), *Melanoma* (LOX IMVI), *Renal Cancer* (UO-31), *Breast Cancer* (T-47D) |
| **5b** | 7.33 | 19/58 | 46.83 to 10.48 | *Leukemia* (CCRF-CEM, HL-60(TB), K-562, MOLT-4, PRMI-8226, SR), *NSC Lung Cancer* (HOP-62, NCI-H226), *CNS Cancer* (SF-268, SF-539, SNB-19, SNB-75), *Melanoma* (LOX IMVI, SK-MEL-5, UACC-62), *Renal Cancer* (A498, UO-31), *Breast Cancer* (T-47D, MDA-MB-468) |
| **6a** | 18.31 | 46/58 | 66.90 to 10.12 | *Leukemia* (CCRF-CEM, HL-60(TB), K-562, MOLT-4, PRMI-8226, SR), *NSC Lung Cancer* (A549/ATCC, HOP-62, NCI-H226, HOP-92, NCI-H23, NCI-H322M, NCI-H460, NCI-H522), *Colon Cancer* (HCC-2998, HCT-116, HCT-15, KM12), *CNS Cancer* (SF-268, SF-295, SF-539, SNB-19,SNB-75, U251), *Melanoma* (LOX IMVI, M14, MDA-MB-435, SK-MEL-2, SK-MEL-5, UACC-62), *Ovarian Cancer* (IGROV1, OVCAR-3, OVCAR-5, OVCAR-8, NCI/ADR-RES), *Renal Cancer* (A498, ACHN, CAKI-1, RXF 393, SN12C, UO-31), *Prostate Cancer* (PC-3), *Breast Cancer* (MDA-MB-2311ATCC, BT-549, T-47D, MDA-MB-468) |
| **6b** | 43.83 | 56/58 | 91.28 to 13.08 | *Leukemia* (CCRF-CEM, HL-60(TB), K-562, MOLT-4, PRMI-8226, SR), *NSC Lung Cancer* (A549/ATCC, HOP-62, NCI-H226, HOP-92, NCI-H23, NCI-H322M, NCI-H460, NCI-H522), *Colon Cancer* (COLO 205, HCC-2998, HCT-116, HCT-15, HT29, KM12, SW-620), *CNS Cancer* (SF-268, SF-295, SF-539, SNB-19,SNB-75, U251), *Melanoma* (LOX IMVI, MALME-3M, M14, MDA-MB-435, SK-MEL-28, SK-MEL-5, UACC-257, UACC-62), *Ovarian Cancer* (IGROV1, OVCAR-4, OVCAR-5, OVCAR-8, NCI/ADR-RES, SK-OV-3), *Renal Cancer* (786-0, A498, ACHN, CAKI-1, RXF 393, SN12C, TK-10, UO-31), *Prostate Cancer* (PC-3, DU-145), *Breast Cancer* (MCF7, MDA-MB-231/ATCC , BT-549, T-47D, MDA-MB-468) |
| **7a** | 93.76 | 58/58 | >100.0 to 40.83 | *Leukemia* (CCRF-CEM, HL-60(TB), K-562, MOLT-4, PRMI-8226, SR), *NSC Lung Cancer* (A549/ATCC, HOP-62, NCI-H226, HOP-92, NCI-H23, NCI-H322M, NCI-H460, NCI-H522), *Colon Cancer* (COLO 205, HCC-2998, HCT-116, HCT-15, HT29, KM12, SW-620), *CNS Cancer* (SF-268, SF-295, SF-539, SNB-19,SNB-75, U251), *Melanoma* (LOX IMVI, MALME-3M, M14, MDA-MB-435, SK-MEL-2, SK-MEL-28, SK-MEL-5, UACC-257, UACC-62), *Ovarian Cancer* (IGROV1, OVCAR-3, OVCAR-4, OVCAR-5, OVCAR-8, NCI/ADR-RES, SK-OV-3), *Renal Cancer* (786-0, A498, ACHN, CAKI-1, RXF 393, SN12C, TK-10, UO-31), *Prostate Cancer* (PC-3, DU-145), *Breast Cancer* (MCF7, MDA-MB-231/ATCC , BT-549, T-47D, MDA-MB-468) |

| | | | | |
|---|---|---|---|---|
| MGI%: Mean growth inhibition percentage. PCE: Positive cytotoxic effect which is ratio between number of cell lines with percentage growth inhibition from 10 to >100 and total number of cell lines. RGI%: Range of growth inhibition percentage. | | | | |

**Table 2. Antitumor activity of the ciprofloxacin and N-alkyl fluoroquinolones at 10µM concentration**

| | | | | |
|---|---|---|---|---|
| 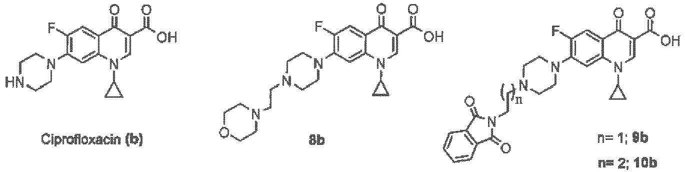 | | | | |

| Compd No. (NCI No) | MGI% | PCE | RGI% | 60 cell lines assay in one dose 10.0 mM concentration Most sensitive cell lines |
|---|---|---|---|---|
| Ciprofloxacin (**b**) | -1.41 | 2/57 | 28.85 to 13.76 | *Renal Cancer* (UO-31, A498) |
| **8b** | -2.20 | 5/57 | 24.51 to 10.05 | *NSC Lung Cancer* (HOP-92, NCI-H522), *Melanoma* ( MALME-3M), *Renal Cancer* (UO-31), *Prostate Cancer* (PC-3) |
| **9b** | -2.69 | 3/58 | 36.83 to 13.18 | *CNS Cancer* (SNB-75), *Renal Cancer* (UO-31), *Breast Cancer* (MCF7) |
| **10b** | -4.16 | 3/57 | 38.76 to 13.64 | *Leukemia* (CCRF-CEM, PRMI-8226), *Renal Cancer* (UO-31) |

| | | | | |
|---|---|---|---|---|
| MGI%: Mean growth inhibition percentage. PCE: Positive cytotoxic effect which is ratio between number of cell lines with percentage growth inhibition from 10 to >100 and total number of cell lines. RGI%: Range of growth inhibition percentage. | | | | |

**Table 3. %Growth inhibition of the most active fluoroquinolones against individual cell lines.**

| *Subpanel tumor cell lines* | *% Growth Inhibition (GI %)^{a}* | | | | | | | |
|---|---|---|---|---|---|---|---|---|
| | **1a** | **2a** | **2b** | **3b** | **6a** | **6b** | **7a** | **5-Flu** |
| *Leukemia* | | | | | | | | |
| CCRF-CEM | >100 | 97.22 | 18.97 | 83.11 | 18.2 | 62.67 | 87.9 | 57.1 |
| HL-60(TB) | 91.04 | >100 | 13.13 | >100 | 26.95 | 91.28 | >100 | 47.9 |
| K-562 | 78.59 | 89.06 | - | 83.06 | 20.11 | 36.45 | 84.58 | 42.3 |
| MOLT-4 | 76.39 | 98.2 | 24.07 | 96.56 | 24.12 | 38.96 | 91.59 | 43.1 |
| PRMI-8226 | 86.57 | 90.93 | 21.26 | 95.14 | 35.93 | 77.65 | 93.53 | 41.4 |
| SR | ND | >100 | 17.66 | >100 | 35.52 | 60.94 | 97.85 | 24.8 |

| *Non-Small Cell Lung Cancer* | | | | | | | | |
|---|---|---|---|---|---|---|---|---|
| A549/ATCC | 84.43 | 97.95 | - | 93.98 | 21.96 | 79.7 | 96.22 | 34.2 |
| HOP-62 | ND | 80.5 | 11.78 | 84.8 | 28.48 | 42.73 | 84.87 | 47.8 |
| NCI-H226 | 39.86 | 86.87 | 39.72 | 58.6 | 23.12 | 28.19 | 78.11 | 69.5 |
| HOP-92 | >100 | >100 | - | 87.8 | 12.64 | 53.06 | >100 | 50.6 |
| NCI-H23 | >100 | >100 | 16.79 | 98.24 | 22.28 | 42.63 | 99.94 | 39.0 |
| NCI-H322M | 54.08 | >100 | 12.84 | 78.73 | 16.46 | 25.29 | >100 | 59.5 |
| NCI-H460 | 94.08 | 89.1 | - | 94.23 | 17.22 | 60.54 | 91.96 | 13.0 |
| NCI-H522 | 91.61 | >100 | - | >100 | 13.46 | 32.68 | >100 | 58.0 |

| *Colon Cancer* | | | | | | | | |
|---|---|---|---|---|---|---|---|---|
| COLO 205 | 87.87 | >100 | 11.94 | >100 | - | 42.44 | >100 | 40.2 |
| HCC-2998 | 75.54 | 69.37 | - | 75.16 | 17.51 | 52.34 | 68.42 | >100 |
| HCT-116 | 95.2 | 93.63 | 13.45 | 96.05 | 20.85 | 67.81 | 94.55 | 17.8 |
| HCT-15 | 84.34 | 92.42 | 16.18 | 93.76 | 28.47 | 44.94 | 88.9 | 26.5 |
| HT29 | 92.14 | 98.13 | - | 99.74 | - | 71.74 | 96.04 | 27.1 |
| KM12 | 95.7 | 90.6 | - | 85.43 | 12.79 | 39.33 | 88.28 | 40.7 |
| SW-620 | 76.04 | 83.65 | - | 79.29 | - | 20.17 | 77.19 | 50.1 |

| *CNS Cancer* | | | | | | | | |
|---|---|---|---|---|---|---|---|---|
| SF-268 | 77.28 | 75.44 | 16.35 | 80.36 | 16.29 | 43.33 | 83.61 | 59.0 |
| SF-295 | 96.06 | >100 | - | >100 | 51.60 | 66.75 | 92.32 | 69.1 |
| SF-539 | 78.3 | 91.42 | - | 88.63 | 20.08 | 53.5 | 82.56 | >100 |
| SNB-19 | 79.39 | 73.77 | - | 75.33 | 10.12 | 33.73 | 71.16 | 65.9 |
| SNB-75 | 58.16 | 25.22 | 70.20 | 86.8 | 29.22 | 63.38 | 85.92 | 65.9 |
| U251 | 87.97 | 87.91 | - | 87.56 | 16.65 | 80.32 | 85.77 | 50.3 |

| *Melanoma* | | | | | | | | |
|---|---|---|---|---|---|---|---|---|
| LOX IMVI | 80.12 | >100 | 38.57 | 98.79 | 38.72 | 70.39 | >100 | 30.4 |
| MALME-3M | 76.25 | >100 | - | >100 | - | 40.29 | >100 | 58.2 |
| M14 | 81.1 | 84.12 | - | 81.47 | 24.44 | 36.45 | 82.14 | Nt |
| MDA-MB-435 | 74.9 | 87.23 | - | 87.01 | 10.88 | 23.91 | 88.61 | 36.6 |
| SK-MEL-2 | 56.19 | >100 | - | >100 | 11.31 | - | 91.18 | 95.5 |
| SK-MEL-28 | 55.42 | - | 49.08 | 51.5 | - | 30.87 | 40.83 | Nt |
| SK-MEL-5 | 73.4 | >100 | 79.75 | 97.94 | 20.46 | 45.06 | >100 | 33.7 |
| UACC-257 | 70.99 | >100 | - | 91.13 | - | 13.08 | 85.98 | 19.5 |
| UACC-62 | 78.52 | 83.85 | 14.58 | 70.38 | 24.14 | 23.40 | 68.78 | 39.7 |

| *Ovarian Cancer* | | | | | | | | |
|---|---|---|---|---|---|---|---|---|
| IGROV1 | 90.63 | 92.5 | - | 70.7 | 20.62 | 64.31 | 86.31 | 51.2 |
| OVCAR-3 | 71.75 | 92.11 | - | 90.13 | 10.76 | - | >100 | 47.4 |
| OVCAR-4 | 62.33 | - | 35.94 | 79.24 | - | 24.39 | 76.15 | 59.4 |
| OVCAR-5 | 48.09 | 92.08 | - | 87.52 | 11.41 | 57.79 | 84.54 | 44.3 |
| OVCAR-8 | 87.42 | 94.16 | - | 94.52 | 18.51 | 40.52 | 91.57 | Nt |
| NCI/ADR-RES | 31.3 | 90.66 | - | 46.49 | 15.18 | 30.14 | 78.65 | 47.6 |
| SK-OV-3 | 59.23 | 98.3 | - | 73.19 | - | 18.39 | 84.38 | 77.5 |

| *Renal Cancer* | | | | | | | | |
|---|---|---|---|---|---|---|---|---|
| 786-0 | 76.35 | 81.27 | - | 72.2 | - | 24.42 | 75.73 | 48.7 |
| A498 | >100 | >100 | 23.58 | 86.25 | 21.15 | 37.03 | >100 | >100 |
| ACHN | 71.35 | >100 | 14.87 | 95.05 | 24.24 | 48.7 | >100 | 39.3 |
| CAKI-1 | 56.36 | 95.25 | 12.12 | 96.94 | 10.66 | 32.91 | 99.06 | 39.4 |
| RXF 393 | 86.6 | 72.06 | 67.74 | 70.95 | 11.11 | 48.66 | 70.98 | 34.3 |
| SN12C | 88.39 | 89.73 | 13.79 | 89.63 | 11.46 | 86.08 | 88.09 | 54.0 |
| TK-10 | 46.4 | 87.63 | - | 66.81 | - | 13.54 | 74.01 | 66.9 |
| UO-31 | 48.92 | >100 | 40.04 | 89.98 | 66.90 | 83.86 | >100 | 41.3 |

| *Prostate Cancer* | | | | | | | | |
|---|---|---|---|---|---|---|---|---|
| PC-3 | 74.74 | 98.4 | 18.91 | 83.79 | 38.94 | 32.08 | 92.18 | 58.2 |
| DU-145 | 68.32 | 88.44 | - | 82.81 | - | 28.56 | 81.12 | 35.5 |

| *Breast Cancer* | | | | | | | | |
|---|---|---|---|---|---|---|---|---|
| MCF7 | 85.71 | 10.75 | 82.38 | 86.55 | - | 46.0 | 85.4 | 11.5 |
| MDA-MB- 231/ATCC | 58.88 | 74.46 | - | 63.36 | 16.33 | 15.37 | 76.08 | 78.1 |
| HS 578T | 81.1 | ND | NT | ND | NT | ND | ND | >100 |
| BT-549 | 87.41 | >100 | 13.68 | >100 | 26.26 | 22.67 | >100 | 37.8 |
| T-47D | 58.92 | 98.3 | 26.38 | 83.7 | 25.94 | 37.05 | 94.71 | 56.7 |
| MDA-MB-468 | ND | >100 | 65.11 | >100 | 19.73 | 34.85 | 95.21 | Nt |

**Table 4. Average antitumor activity of compounds 1a, 2a, 3b, 6b and 7a against tumor cell lines from nine different organs at 10-fold dilution of five concentrations; median growth inhibitory (GI₅₀, µM), total growth inhibitory (TGI, µM) and median lethal (LC₅₀, µM)**

| Compd. | Subpanel tumor cell lines | | | | | | | | | | MG-MID^{a} |
|---|---|---|---|---|---|---|---|---|---|---|---|
| | Activity | leukemia | NSC lung cancer | colon cancer | CNS cancer | melanoma | ovarian cancer | renal cancer | prostate cancer | breast cancer | |
| **1a** | GI₅₀ | 2.89 | 2.87 | 3.38 | 3.57 | 2.56 | 3.16 | 2.93 | 3.21 | 2.95 | 2.95 |
| | TGI | 80.96 | 53.38 | 73.15 | 70.26 | 29.36 | 54.29 | 54.00 | 54.11 | 54.63 | 29.51 |
| | LC₅₀ | c | c | 95.24 | c | 89.65 | c | c | c | c | 95.49 |
| **2a** | GI₅₀ | 3.07 | 2.49 | 3.15 | 3.03 | 2.30 | 2.96 | 2.73 | 2.62 | 2.51 | 2.63 |
| | TGI | 62.54 | 19.61 | 72.89 | 34.84 | 6.36 | 39.54 | 23.97 | 8.72 | 8.44 | 14.12 |
| | LC₅₀ | c | 73.15 | 77.21 | 77.16 | 39.48 | 76.67 | 82.47 | c | 88.06 | 57.54 |
| **3b** | GI₅₀ | 3.02 | 3.07 | 3.79 | 3.68 | 2.54 | 3.39 | 3.19 | 3.29 | 3.04 | 3.09 |
| | TGI | 62.55 | 84.28 | 87.41 | c | 52.41 | 88.90 | 87.07 | c | 81.37 | 60.25 |
| | LC₅₀ | c | c | c | c | 87.08 | c | c | c | c | 95.49 |
| **6b** | GI₅₀ | 2.57 | 2.88 | 3.46 | 2.95 | 22.31 | 2.89 | 2.78 | 3.40 | 2.85 | 3.01 |
| | TGI | 76.70 | c | c | c | c | c | c | c | 76.89 | 89.12 |
| | LC₅₀ | c | c | c | c | c | c | c | c | c | c |
| **7a** | GI₅₀ | 3.48 | 3.04 | 3.39 | 3.09 | 3.20 | 3.07 | 3.50 | 3.71 | 2.81 | 3.09 |
| | TGI | 63.22 | 46.59 | 67.85 | 63.40 | 28.33 | 62.35 | 63.09 | c | 38.49 | 29.51 |
| | LC₅₀ | c | c | 86.79 | c | 99.66 | c | c | c | c | 95.49 |
| **5-FU^{b}** | GI₅₀ | 15.1 | c | 8.4 | 72.1 | 70.6 | 61.4 | 45.6 | 22.7 | 76.4 | 22.60 |
| | TGI | c | c | c | c | c | c | c | c | c | c |
| | LC₅₀ | c | c | c | c | c | c | c | c | c | c |

| | | | | | | | | | | | |
|---|---|---|---|---|---|---|---|---|---|---|---|
| ^{a}Full panel mean-graph midpoint (µM). c: Compounds showed values > 100µM. ^{b}5-fluorouracil as reference drug | | | | | | | | | | | |

**Table 5. Influence of compounds 1a, 2a, 3b, 6b and 7a on the growth of the individual tumor cell lines; median growth inhibitory (GI₅₀, µM)**

| *Subpanel tumor cell lines* | GI₅₀ (*µ*M) | | | | |
|---|---|---|---|---|---|
| | **1a** | **2a** | **3b** | **6b** | **7a** |
| *Leukemia* | | | | | |
| CCRF-CEM | 2.93 | 3.03 | 2.61 | 2.80 | 3.67 |
| HL-60(TB) | 2.18 | 2.22 | 2.04 | Nt | 2.58 |
| K-562 | 2.91 | 4.00 | 3.92 | Nt | 3.93 |
| MOLT-4 | 3.83 | 3.55 | 4.00 | Nt | 4.46 |
| PRMI-8226 | 3.08 | 2.55 | 2.53 | 2.34 | 2.79 |

| *Non-Small Cell Lung Cancer* | | | | | |
|---|---|---|---|---|---|
| A549/ATCC | 2.81 | 2.89 | 3.08 | 2.23 | 2.87 |
| HOP-62 | 4.47 | 3.08 | 4.77 | 3.65 | 4.6 |
| NCI-H226 | 3.02 | 2.60 | 3.58 | 1.23 | 3.71 |
| HOP-92 | Nt | 1.22 | 1.54 | 1.52 | 1.25 |
| NCI-H23 | 2.67 | 2.33 | 2.90 | 3.41 | 3.13 |
| NCI-H322M | Nt | 2.86 | 3.24 | 5.19 | 2.6 |
| NCI-H460 | 3.16 | 3.13 | 3.66 | 3.04 | 3.43 |
| NCI-H522 | 1.73 | 1.81 | 1.99 | 2.80 | 2.78 |

| *Colon Cancer* | | | | | |
|---|---|---|---|---|---|
| COLO 205 | 2.62 | 2.44 | 3.50 | 3.40 | 2.02 |
| HCC-2998 | 4.10 | 2.29 | 4.47 | 5.37 | 4.26 |
| HCT-116 | 3.18 | 3.56 | 3.86 | 2.98 | 3.88 |
| HCT-15 | 3.62 | 2.82 | 3.30 | 2.55 | 3.15 |
| HT29 | 2.64 | 3.63 | 3.77 | 3.15 | 3.95 |
| KM12 | 4.07 | 3.42 | 3.62 | 3.38 | 2.96 |
| SW-620 | 3.43 | 3.91 | 4.05 | 3.40 | 3.57 |

| *CNS Cancer* | | | | | |
|---|---|---|---|---|---|
| SF-268 | 3.85 | 3.86 | 4.77 | 3.96 | 4.39 |
| SF-539 | 4.12 | 1.92 | 3.39 | 3.14 | 2.43 |
| SNB-19 | 3.97 | 3.07 | 3.25 | 2.56 | 3.47 |
| SNB-75 | 2.60 | Nt | Nt | 2.23 | 1.89 |
| U251 | 3.10 | 3.30 | 3.33 | 2.89 | 3.29 |

| *Melanoma* | | | | | |
|---|---|---|---|---|---|
| LOX IMVI | 2.40 | 1.48 | 1.44 | 1.42 | 1.63 |
| MALME-3M | 1.99 | 1.95 | 2.30 | Nt | 2.83 |
| M14 | 2.85 | 3.24 | 4.06 | 4.69 | 4.37 |
| MDA-MB-435 | 2.47 | 1.97 | 2.97 | Nt | 3.13 |
| SK-MEL-2 | 2.57 | 2.75 | 3.16 | Nt | 3.55 |
| SK-MEL-28 | 4.88 | 3.21 | 2.67 | Nt | 6.09 |
| SK-MEL-5 | 1.80 | 1.87 | 2.04 | 3.03 | 2.24 |
| UACC-257 | 2.16 | 2.65 | 2.48 | >100 | 2.72 |
| UACC-62 | 2.02 | 1.61 | 1.75 | 2.43 | 2.25 |

| *Ovarian Cancer* | | | | | |
|---|---|---|---|---|---|
| IGROV1 | 3.14 | 2.33 | 2.73 | 2.66 | 2.47 |
| OVCAR-3 | 3.51 | 2.54 | 3.90 | 3.17 | 3.69 |
| OVCAR-4 | 2.20 | 2.58 | 2.28 | 2.37 | 2.60 |
| OVCAR-5 | 4.25 | 3.43 | 4.14 | Nt | 3.38 |
| OVCAR-8 | 2.80 | 3.30 | 3.33 | 2.87 | 3.09 |
| NCI/ADR-RES | 2.98 | 3.28 | 4.01 | 3.29 | 3.20 |
| SK-OV-3 | 3.28 | 3.00 | Nt | 3.02 | 3.11 |

| *Renal Cancer* | | | | | |
|---|---|---|---|---|---|
| 786-0 | 3.77 | 3.19 | 4.02 | 4.08 | 4.50 |
| A498 | 1.81 | 2.99 | 2.91 | 2.37 | 2.74 |
| ACHN | 3.09 | 2.62 | 3.10 | 2.46 | 2.88 |
| CAKI-1 | 2.75 | 2.01 | 2.71 | 1.80 | 2.33 |
| RXF 393 | 3.32 | 2.08 | 2.98 | 2.41 | 3.19 |
| SN12C | 2.92 | 2.77 | 2.97 | 2.61 | 2.59 |
| TK-10 | 3.73 | 4.02 | 4.31 | 4.57 | 7.60 |
| UO-31 | 2.07 | 2.20 | 2.58 | 1.96 | 2.21 |

| *Prostate Cancer* | | | | | |
|---|---|---|---|---|---|
| PC-3 | 2.31 | 2.43 | 2.46 | NT | 2.66 |
| DU-145 | 4.11 | 2.81 | 4.31 | 3.40 | 3.97 |

| *Breast Cancer* | | | | | |
|---|---|---|---|---|---|
| MCF7 | 2.97 | 2.95 | 2.84 | 2.59 | 2.72 |
| MDA-MB-231/ATCC | 2.51 | 1.88 | 2.14 | 2.03 | 1.91 |
| HS 578T | 4.52 | 2.53 | 4.26 | 2.40 | 2.68 |
| BT-549 | 2.44 | 2.64 | 2.98 | 3.34 | 3.7 |
| T-47D | 3.20 | 3.11 | 3.77 | 4.81 | 3.35 |
| MDA-MB-468 | 2.10 | 1.98 | 2.25 | 1.97 | 2.52 |

## Claims

1. Compound of general formula (I): 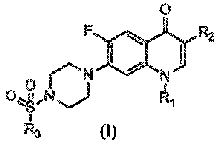
wherein R₁ is cyclopropyl;
R₂ is a carboxyl group; and ;
R₃ is selected from 4-chlorophenyl, 2,4-dichlorophenyl, 3,4-dichlorophenyl, 3,4-dimethoxyphenyl, 2,4-dimethoxyphenyl, and 2,4,6-trimethylphenyl;
or pharmaceutically acceptable salt thereof for use in treating cancer.

## Patentansprüche

1. Verbindung der allgemeinen Formel (I): 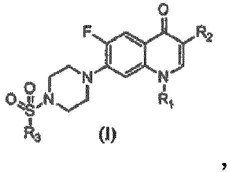
wobei R₁ Cyclopropyl ist;
R₂ eine Carboxylgruppe ist; und
R₃ ausgewählt ist aus 4-Chlorphenyl, 2,4-Dichlorphenyl, 3,4-Dichlorphenyl, 3,4-Dimethoxyphenyl, 2,4-Dimethoxyphenyl und 2,4,6-Trimethylphenyl;
oder ein pharmazeutisch akzeptables Salz davon zur Verwendung in der Behandlung von Krebs.

## Revendications

1. Composé de la formule générale (I): 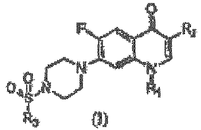
dans laquelle R₁ représente le cyclopropyle ;
R₂ représente un groupe carboxyle ; et ;
R₃ est sélectionné parmi le 4-chlorophényle, le 2,4-dichlorophényle, le 3,4-dichlorophényle, le 3,4-diméthoxyphényle, le 2,4-diméthoxyphényle et le 2,4,6-triméthylphényle ;
ou un sel pharmaceutiquement acceptable de ceux-ci pour une utilisation dans le traitement du cancer.
